# EUROPEAN PATENT APPLICATION

(11) **EP 4 826 385 A1**
(43) Date of publication of application: **21.10.2026**
(21) Application number: 25170757.6
(22) Date of filing: 15.04.2025
(51) Int. Cl.: G01N 33/569, G01N 33/68

(54) **METHODS FOR PREDICTING RISK FOR INFLAMMATION**

(71) Applicant: MK Capital Invest AB, 222 29 Lund (SE)
(72) Inventor: Sjögren, Marie, 247 72 Genarp (SE); Tariq, Mohammad, Pittsburgh, PA 15213 (US)
(74) Representative: KIPA AB

(57) **Abstract**

The present invention relates to method of determining risk of an inflammatory condition, and/or risk of progression of an inflammatory condition, as well as to compounds for use in these methods.

## Description

### Technical field

The present invention relates to methods of determining risk of an inflammatory condition, and/or risk of progression of an inflammatory condition, as well as to compounds for use in these methods.

### Background

Inflammation is a critical physiological response implicated in a wide range of conditions, including autoimmune disorders, infectious diseases, and chronic illnesses such as cardiovascular, kidney and Alzheimer's disease. Accurate detection and monitoring of inflammation levels play an essential role in both diagnosing these conditions and guiding treatment decisions.

Conventional approaches, such as blood tests that measure markers like C-reactive protein (CRP) or erythrocyte sedimentation rate (ESR), although they show a general health conditions, can lack the specificity or sensitivity necessary for early disease detection or nuanced evaluation of a patient's inflammatory status. Additionally, more advanced molecular diagnostics often require complex, expensive laboratory equipment and may not provide rapid, point-of-care results.

Given these challenges, there is a growing need for more precise, reliable, and readily deployable methods to quantify inflammatory activity. Improved diagnostic techniques can help clinicians better stratify disease severity, tailor therapeutic interventions, and assess patient response to treatment in real time. By addressing the limitations of existing diagnostic protocols-such as cost, invasiveness, and limited resolution of current assays-innovations in inflammation and disease detection can lead to more personalized patient care and potentially better clinical outcomes.

### Summary

The inventor has surprisingly discovered that mitochondrial voltage-dependent anion channel 1 (VDAC1), is an early predictor of inflammatory conditions in human subjects, including Type 1 Diabetes, Cardiovascular plaque formation, kidney diseases, and also severity of inflammatory conditions. The VDAC1 detection in could also predict the initiation/progression of Alzheimer's disease. Thus, VDAC1 is an early marker of inflammation in a subject. If the subject has a co-existing inflammatory condition, severity of inflammation indicates the progression of that inflammatory condition.

The present invention provides *in vitro* method for determining risk for an inflammatory condition, and/or risk of progression of an inflammatory condition in a subject, compositions for use in said method, as well as to further methods.

The invention in one aspect provides an *in vitro* method for determining level of inflammation in a subject, comprising
a. Detecting the level of VDAC1 in a biological sample from the subject, and
b. Comparing the level in sample to control.

Related to this aspect, the invention provides an *in vitro* method for determining risk of an inflammatory condition in a subject, comprising a) Detecting the level of VDAC1 in a biological sample from the subject, and b) Comparing the level in sample to control.

The invention further provides an *in vitro* method for determining risk of progression of an inflammatory condition in a subject determined to be at risk of or diagnosed with an inflammatory condition, comprising a) Detecting the level of VDAC1 in a biological sample from the subject, and b) Comparing the level in sample to control.

In another aspect, the invention provides an antigen-binding polypeptide having binding specificity for VDAC1 for use in a diagnostic method practiced on the human or animal body, wherein the antigen-binding polypeptide is selected from the group consisting of an antibody, an antigen-binding fragment thereof, and any variants, fusions, or derivatives thereof.

In a further aspect, the invention provides an antigen-binding polypeptide having binding specificity for VDAC1 for use in the *in vitro* method according to the invention, the polypeptide being selected from the group consisting of an antibody, an antigen-binding fragment thereof, and any variants, fusions, or derivatives thereof.

In a yet further aspect, the invention provides a compound for use in treatment of an inflammatory condition in a subject, wherein the subject is determined to be at risk for an inflammatory condition, or at risk for progression of an inflammatory condition, by the method according to the invention.

In a yet further aspect, the invention provides a method of determining a treatment regimen comprising (1) performing the *in vitro* method according to the invention and (2) determining a treatment protocol based on the determination.

### Description of drawings

- **Fig 1.**: VDAC1 protein measured by ELISA in serum samples from newly diagnosed Type I Diabetes subjects compared to non-diabetic controls (A). VDAC1 protein measured by ELISA in plasma samples from Type I Diabetes-prone subjects with 1, 2 or 3 antibodies compared subjects without antibodies (B). See Examples 1 and 2.
- **Fig 2.**: VDAC1 expression in pancreatic islets from pancreatic sections taken from T 1D-prone NOD mice. See Example 3
- **Fig 3.**: VDAC1 secretion to culture medium from kidney podocytes is increased in presence of cytokine mixture (IL-1β: 1 ng/ml, TNF-α: 250 ng/ml, INF-y: 250 ng/ml). See Example 4.
- **Fig 4.**: Increased VDAC1 level in serum correlates with viral infections and disease progression and severity. See Example 5.
- **Fig 5.**: Increased VDAC1 level in serum in plasma from mice fed a high fat diet (HFD) as compared to normal diet (ND).

### Detailed description

### Methods

The invention relates in the first aspect to an *in vitro* method for determining risk of an inflammatory condition in a subject, comprising
a. Detecting the level of VDAC1 in a biological sample from the subject,
b. Comparing the level in sample to control.

The invention also relates to an *in vitro* method for determining risk of progression of an inflammatory condition in a subject determined to be at risk of, or diagnosed with, an inflammatory condition; said method comprising detecting the level of VDAC1 in a biological sample from the subject, and comparing the level in sample to control.

A further embodiment of the invention relates to the *in vitro* method according to the invention wherein the method is for determining the risk of more severe progression.

The invention further relates to an *in vitro* method for determining the level of inflammation a subject, comprising detecting the level of VDAC1 in a biological sample from the subject, and optionally comparing the level in sample to control.

According to the methods of the invention, a level of VDAC1 which is higher than control indicates increased risk for an inflammatory condition and/or progression of the inflammatory condition.

### Inflammation

Inflammation is the body's natural response to injury, infection, or other irritant, aimed at eliminating the harmful agent and/or initiating the healing process.

While inflammation may be the first step in a healing process in normal circumstances, the level of inflammation response may vary greatly in in different subjects.

Increased levels of inflammation may be in themselves problematic but are also likely to contribute to progression of an inflammatory condition.

The term "inflammatory condition" as used herein refers to any disease or condition which comprises inflammation, such as established inflammatory diseases, or other conditions where inflammation forms part of the clinical presentation.

The term "progression" of an inflammatory condition in this context refers to worsening of clinical symptoms, e.g. one or more of increased pain, decreased mobility, decreased function of organs. Progression as used herein may also comprise development of additional complications or pathologies, as well as accelerated development of symptoms/ complications. For example, chronic illnesses typically progress over time. The expression "more severe progression" indicates an accelerated course of disease, i.e. faster progression; and/or progression where the worsening is more than average, e.g. increased pain, more additional complications or pathologies than in a typical course of disease.

Early indications of the level of inflammation likely to develop is valuable for informing choices regarding treatment.

In some embodiments, the invention relates to the *in vitro* method according to the invention wherein the inflammatory condition is a chronic inflammatory disease or a condition with acute inflammatory component.

The term "chronic inflammatory disease" refers to a long-lasting medical condition characterized by persistent or recurring inflammation in the body.

Chronic inflammatory disease may for example be one or more of the following: rheumatoid arthritis, inflammatory bowel disease (such as Crohn's disease and ulcerative colitis), psoriasis, chronic obstructive pulmonary disease (COPD); cystic fibrosis, diabetes, such as e.g. Type I diabetes or Type II diabetes; kidney disease; neurodegenerative disease such as for example Alzheimer's; cardiovascular disease such as for example atherosclerotic plaque; cerebrovascular disease; autoimmune disease; and low-grade inflammation associated with aging.

Particular embodiments relate to an *in vitro* method according to the invention, wherein the chronic inflammatory disease is Type I Diabetes (T1D).

In some embodiments the inflammatory condition is a disease or condition with acute inflammatory component.

The term "acute inflammatory component" refers to a medical condition characterized by a rapid onset of inflammation that typically lasts for a relatively short duration (days to a few weeks), but which may in dysregulated conditions become extended.

In one embodiment, the disease or condition with an acute inflammatory component is is selected from the group consisting of infections, such as viral infection, for example COVID 19; trauma; and sepsis.

### VDAC1

VDAC1 (Voltage-Dependent Anion Channel 1) is a mitochondrial protein primarily located in the outer mitochondrial membrane. VDAC1 is implicated in regulating the exchange of metabolites and ions between the mitochondria and the cytoplasm. VDAC1 is involved in various cellular processes, including energy production, cell signalling, and apoptosis.

The methods of the invention comprise detecting the level of VDAC1 in a biological sample from the subject.

Detecting the level of VDAC1 in a biological sample may be done in any suitable manner. For example, the level of the VDAC1 may comprise one or more of detecting the amount of polypeptide or fragments thereof, detecting the expression of nucleic acid encoding the polypeptide, detecting the amount of mRNA for polypeptide or its complement expressed from the gene encoding VDAC1.

### Anti VDAC1- antibodies

Preferably, detecting the level of VDAC1 is by detecting the level of VDAC1 polypeptide in a biological sample. This may be done by any suitable method such as for example by contacting the sample with an antigen-binding polypeptide having binding specificity for VDAC1, the polypeptide being selected from the group consisting of an antibody, an antigen-binding fragment thereof, and any variants, fusions, or derivatives thereof.

Thus, some embodiments relate to the methods according to the invention wherein detecting the level of VDAC1 comprises contacting the sample with an antigen-binding polypeptide having binding specificity for VDAC1, the polypeptide being selected from the group consisting of an antibody, an antigen-binding fragment thereof, and any variants, fusions, or derivatives thereof.

Particular embodiments relate to wherein detecting the level of VDAC1 comprises contacting the sample with an antibody with specificity for VDAC1.

Antibodies suitable for detecting VDAC1 may be generated by methods known in the art.

For example, the detection of VDAC1 may be done by means of an assay selected from the group comprising lateral flow immunoassay (LFIA, aka dipstick test), immunochromatographic strip test, latex agglutination test (LAT), point-of-care immunosensors or biosensors, microfluidic-based rapid immunoassays, "lab-on-a-chip" devices integrating sample handling, mixing, and detection, flow-through immunoassay (e.g. dot-blot or spot test), immunoturbidimetric and nephelometric methods, snap tests (enzyme-linked immunosorbent assay in a cartridge) and enzyme-linked immunoassay (ELISA).

### Biological sample

The biological sample may be any suitable sample, such as one or more of cells, tissue, blood, serum, plasma, urine, cerebrospinal fluid, pancreatic fluid or cell-culture medium. Preferably, the biological sample according to the method is selected from serum, plasma, blood, cell culture medium or any combination thereof.

Particular embodiments relate to wherein the biological sample is serum, plasma or blood. These samples have the advantage of being readily available and minimally invasive. Furthermore, such samples are routinely taken in connection with determining risk of diagnosis of an inflammatory condition. Performing the *in vitro* method of the invention on these samples will further indicate the risk of progression of said inflammatory condition and/or severity of inflammation.

In some embodiments the method according to the invention relates to biological samples that have already been retrieved from the patient and stored.

### Detecting level of polypeptide in blood

The inventors have surprisingly noted that levels of VDAC1 polypeptide detected in these samples are indicative of later inflammation in subject. Due to VDAC1 mistargeting to the cell surface and its consequent loss from the cells and leakage to blood circulation, levels in extracellular or interstitial fluids are well -suited as biological sample. Thus, particular embodiments relate to where the method of the invention comprises detecting the level of VDAC1 polypeptide in a biological sample selected from the group comprising blood, serum, plasma, cell culture medium or combinations thereof from the subject.

VDAC1 polypeptide detection may be made by any suitable antibody-based methods as mentioned herein above.

### Patient / subject

The terms "patient" and "subject" are used interchangeably herein, and refer to any mammal, in particular a human.

In one embodiment, the present invention relates to where in the subject has been determined to be at risk for, and/or diagnosed with, an inflammatory condition.

The level of inflammation often influences the severity of disease and or its progression. Therefore, some embodiments of the invention relate to where in the subject has an inflammatory condition.

Particular embodiments relate to wherein the subject is at risk for, or has been diagnosed with, Type 1 Diabetes. Thus, in one embodiment relates to the *in vitro* method according to the invention wherein the chronic inflammatory disease is Type I Diabetes.

### Controls

The methods of the invention may comprise a step of comparing the level VDAC1 detected in biological sample to control.

The control may be any suitable control subject or group. Selection of an appropriate control is within the skill of the art. For example, the control may be healthy control subject. The control may be a subject determined to be healthy, or to not have an inflammatory condition, by any suitable means relevant to the patient. For example, healthy might be defined as not displaying the symptoms and/or not fulfilling the requirements for a diagnosis of the disease for which risk is being assessed, such as T1D. The control subject(s) may further be age-matched, sex-matched, matched for lifestyle or for any other one or more conditions or parameters known to influence an inflammatory condition. Alternatively, the control may be a random population sample,

In some embodiments the control may be a subject (s) diagnosed with a condition but who has shown milder (less severe) clinical outcomes.

### Levels/ Risk

The inventors have demonstrated that increased levels of VDAC1 correlate with the severity of inflammatory condition at later stages.

Thus, increased levels of VDAC1 in patients compared to control subjects suggest that the inflammation will be more pronounced in these patients. For example, a patient may be determined to have an elevated risk of inflammation or progression of an inflammatory condition if the level of VDAC1 in a sample from the patient is higher than in a sample from a control subject.

A level considered higher may be for example at least 10%, 20% 30% higher compared to controls, such as 35%, 40%, 55%, or 60% higher.

In some embodiments, a level of VDAC1 in a subject which exceeds the average level of a control (e.g. a that of a population control), is indicative of a risk for an inflammatory condition and/or risk of progression of an inflammatory condition.

If levels of VDAC1 in a subject are substantially similar compared to controls, this may be indicative of a normal level of inflammation, i.e. lower risk for an inflammatory condition and/or no, mild or normal progression of an inflammatory condition.

A level may be considered "substantially similar" if it falls within about 30% variance from the control levels; for instance, within 25%, within 20%, or within 15% variance from the control.

Levels of VDAC1 may also be compared to subjects known or observed to have suffered a more several inflammation/ progressions of inflammatory condition.

Thus, if a patient's levels of VDAC1 are substantially similar to those observed in controls known to experience severe inflammation, it is indicative of risk of severe inflammation, i.e increased risk for an inflammatory condition, and/or risk for progression or severe progression of an inflammatory condition.

Risk may be expressed in various ways, including as a relative value compared with a control, such as a 1.5-fold or 2-fold increase. Alternatively, risk may be expressed as a percentage increase relative to a control. In other embodiments, risk may be expressed as an absolute number, for example, lifetime risk, representing the overall likelihood of an event occurring over the subject's lifespan.

### Early determination

It is particularly valuable to determine risk for inflammatory condition, and/or progression of an inflammatory condition, as early as possible in life or as early as possible in relation to diagnosis or determining risk of an inflammatory condition.

Thus, some embodiments relate to methods according to the invention whereby the subject is less than 7 years old, such as less than 6, less than 5, or less than 4 years of age; or for example no more than 3 years old.

Particular embodiments relate to wherein the subject is no more than two years old, for example no more than 18 months old, no more than 1 year old.

Further particular embodiments relate to wherein the subject is no more than one year old, and has been determined to be at risk for a chronic inflammatory condition, for example Type I diabetes.

Other embodiments relate to where in the method according to the invention is performed within 2 years of the subject being determined to be at risk for and or being diagnosed with an inflammatory condition, such as a chronic inflammatory condition. For example, the method may be performed within 1 year, 8 months or for example within 6 months being determined to be at risk or being diagnosed with an inflammatory condition.

Particular embodiments relate to wherein the method according to the invention is performed on the same sample as is used for determining whether the subject is at risk for and/or diagnosis of an inflammatory condition

### Antigen-binding polypeptide for use in the methods

The present invention provides in a second aspect to an antigen-binding polypeptide having binding specificity for VDAC1 for use in the *in vitro* method according to the invention, the polypeptide being selected from the group consisting of an antibody, an antigen-binding fragment thereof, and any variants, fusions, or derivatives thereof.

Particular embodiments relate to where the antigen binding polypeptides were used in the *in vitro* method according to the present invention as described herein, is one or more antibodies against VDAC1.

### Compounds for use in the methods

The present invention provides in a third aspect a compound for use in the prevention or treatment of an inflammatory condition in a subject, wherein the subject is determined to be at risk for an inflammatory condition, or at risk for progression of an inflammatory condition, by the method of the invention as described herein.

The compound may be one or more of for example nonsteroidal anti-inflammatory drugs (e.g., ibuprofen, naproxen), corticosteroids (e.g., prednisone, prednisolone, dexamethasone), disease-modifying antirheumatic drugs (traditional DMARDs such as methotrexate, sulfasalazine, hydroxychloroquine, and leflunomide; biologic DMARDs such as TNF inhibitors like adalimumab and etanercept, IL-6 inhibitors, IL-17 inhibitors, and targeted synthetic DMARDs such as JAK inhibitors like tofacitinib and baricitinib), and immunosuppressants (e.g., azathioprine, mycophenolate mofetil, cyclosporine).

Particular embodiments relates to a compound for use wherein the inflammatory condition is Type I Diabetes, and wherein said compound is selected from one or more of the groups consisting of biguanides such as metformin, insulin (including insulin analogs such as insulin glargine, detemir, lispro, aspart, and degludec); a GLP-1 receptor agonist (such as exenatide, liraglutide, dulaglutide, or semaglutide); a SGLT2 inhibitor (such as canagliflozin, dapagliflozin, or empagliflozin); a DPP-4 inhibitor (such as sitagliptin, saxagliptin, linagliptin, or vildagliptin); a sulfonylurea (such as glibenclamide, glipizide, or glimepiride);a thiazolidinedione (such as pioglitazone or rosiglitazone); an immunomodulatory agent (such as an anti-CD3 antibody, interleukin inhibitor, or TNF-α inhibitor); blood pressure drugs; cholesterol lowering drugs; anti-platelet drugs (such as aspirin); and pharmaceutically acceptable salts thereof.

### Treatment regime

In a yet further aspect, the invention provides a method of determining a treatment regimen comprising (1) performing the *in vitro* method according to the invention and (2) determining a treatment protocol based on the determination. Particular embodiments relate to determining treatment regimen where the inflammatory condition is a chronic inflammatory condition as described herein above, in particular, Type I Diabetes.

### Antibody for use

Provided herein is an antigen-binding polypeptide having binding specificity for VDAC1 for use in a diagnostic method practiced on the human or animal body, wherein the antigen-binding polypeptide is selected from the group consisting of an antibody, an antigen-binding fragment thereof, and any variants, fusions, or derivatives thereof.

### Examples

### Example 1 -Type I Diabetes

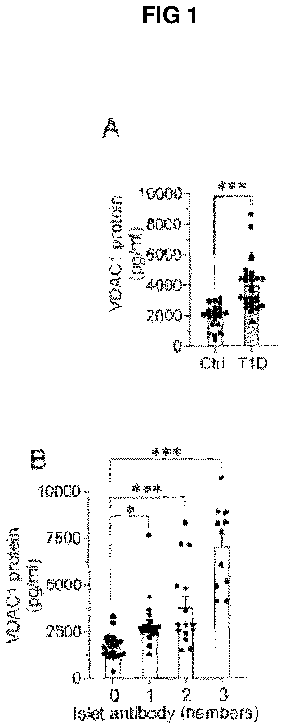

VDAC1 protein was measured by ELISA in serum samples from newly diagnosed Type I Diabetes (T1D) subjects and compared to non-diabetic controls (see Fig 1A).

The patients (n= 45, aged 46 years +- 12 years) were diagnosed with Type I Diabetes . The same samples which were used in diagnosis of T1D were analysed for levels of VDAV1 protein. The results show that T1D patients had significantly higher levels of VDAC1 as compared to controls (Population control, 32 individuals). Type 1 diabetic patients show significantly higher levels of VDAC1 as compared to controls.

### Example 2 -Type I Diabetes

VDAC1 protein was measured by ELISA in plasma samples from Type I Diabetes-prone subjects with 1, 2 or 3 antibodies selected from GADA, IAA, IA2A, ZnT8. compared subjects without these antibodies (see Fig 1B).

Seventy six children aged from 11 to 15 years old were determined to have antibody against HLA-DRA1 and therefore considered to be at risk for Type I diabetes. At the time of sampling the children were considered healthy, having i.a. normal blood sugar levels. Samples from these children were tested for presence of one or more of the following antibodies: Glutamic acid decarboxylase antibodies (GADA), Insulin autoantibodies (IAA), Islet Antigen-2 Antibodies (IA-2A) and Zinc Transporter 8 antibodies (ZnT8) and grouped according to how many of these antibodies were present.

The experiments show that presence of increasing numbers (several types) of antibodies correlates with increasing levels of VDAC1 protein in plasma. (see Fig 1B).

### Example 3- Mouse model

VDAC1 expression in pancreatic islets from pancreatic sections taken from T1D-prone NOD mice were studied.

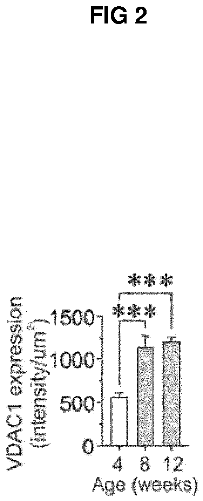

The results show that VDAC1 expression increases over time in T1D-prone mice, correlating with islet degradation (see Fig 2).

### Example 4- Kidney damage

To better understand the causes underlying diabetic kidney complications and to develop a diagnostic test we used kidney podocyte cells. These human podocytes offer the best *in vitro* cell systems to integrate into the investigation of kidney dysfunction. Podocytes were cultured for 24 hours in the presence of indicated agents, thereafter an aliquot of culture medium was removed for the detection of VDAC1 protein released in the medium.

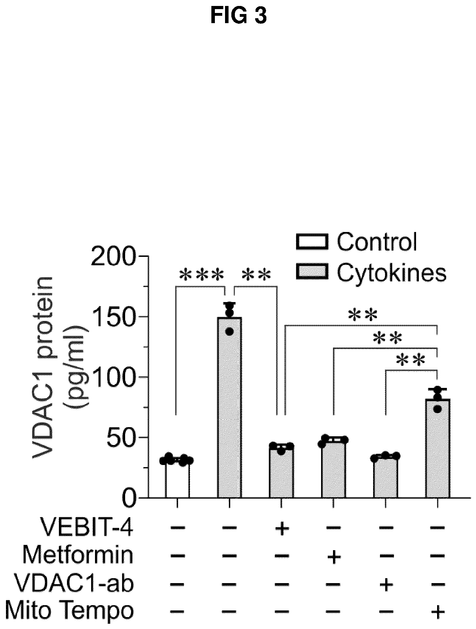

The presence of a cytokine mixture (IL-1β: 1 ng/ml, TNF-α: 250 ng/ml, INF-y: 250 ng/ml) during the culture period significantly increased the secreted/leakage of VDAC1 protein into the culture medium (Fig 3).

### Example 5- COVID-19

### Material and methods

**Patients**-The study cohort consisted of 40 hospitalized patients (>18 years of age) who were admitted to the hospital due to respiratory symptoms for COVID-19 and were positively tested for SARS-CoV-2 (confirmed by RT-PCR). Patients were divided into 3 groups; Group 1 (mild symptoms and no oxygen supplementation), Group 2 (moderate symptoms and 1 to 6 L of oxygen supplementation), and Group 3 (severe symptoms and intensive care was needed). Peripheral blood collected in BD Vacutainer serum tubes was set to coagulate from COVID-19 patients upon enrolment in the hospital at day 0, and at days 3, 7, 10, 14, and 28 for isolation of serum. Serum was prepared by centrifugation at 150 x g for 10 minutes and stored at -80°C until further use. For patients who were discharged from the hospital during the study period, no blood samples were obtained until the follow-up consultation on day 28. Clinical characteristics were compared between severe, moderate and mild cases and the VDAC1 level in the serum was compared to the serum from non-infected healthy donors.

**VDAC1 measurement-** VDAC1 protein was determined in serum and cell homogenates or cell culture medium with Human VDAC1/PORIN ELISA (LSBio) according to manufacturer's instructions. The quantity of cell protein in cell culture experiments was determined by Pierce BCA Protein Assay Kit (#23225; Thermo Fisher Scientific) according to the manufacturer's protocol. VDAC1 values in the cell medium were normalized to the amount of cell protein in each sample.

**Statistical analysis-** Statistical analyses were performed using Graphpad Prism (GraphPad Software Inc, version 9, San Diego, CA, USA). The results are expressed as means±SEM for the indicated number of observations or illustrated by a representative observation of the result obtained from different experiments (confocal microscopy). Differences between two groups were analyzed using Student's t-test and for multiple groups one-way ANOVA followed by Tukey's Dunnett's multiple comparisons test was used. P-value <0.05 was considered significant.

### Results and discussion

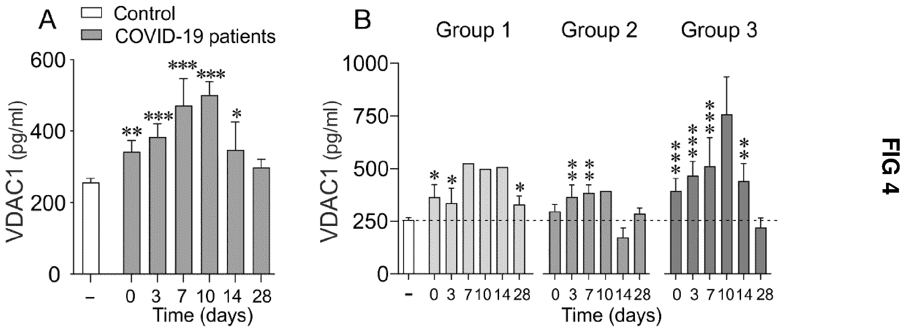

VDAC1 correlates with severity and clinical parameters in COVID-19 patients. VDAC1 expression was analysed in serum isolated from COVID-19 patients. As shown in Fig 3, VDAC1 level in the serum was increased during the progression of the COVID-19 infection (Fig. 4A), with its highest peak at day 10. On day 28 VDAC1 levels in recovered COVID-19 patients was declined to the basal level seen in non-infected controls (Fig. 4A). The magnitude of VDAC1 levels was also dependent on the severity of the COVID-19 infection (Fig 4B). Thus, highest serum level of VDAC1 was observed in group 3, representing patients with severe COVID-19 symptoms (Figure 4B).

### Example 6- VDAC1 is found in serum of cardiovascular plaque in mice

### Materials and methods

VDAC1 (AB_2564843, ab135585), antibodies were purchased from Abcam (Cambridge, UK).

### Results and discussion

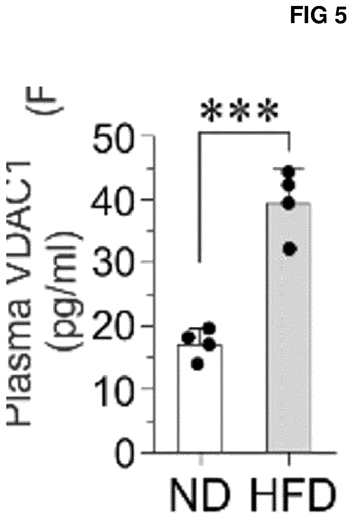

The Apoe-/- mice known to rapidly develop mitochondrial dysfunctionality and controls were subjected to 12 weeks of high fat diet (HFD). Confocal microscopy analysis relative to a membrane marker of EC (CD31) revealed a markedly increased cell surface translocation of VDAC1 in the cardiac sections of HFD-treated Apoe-/-. The HFD mice also displayed a markedly increased plasma level of VDAC1, possibly due to the leakage of VDAC1 from ECs to the blood. See Fig 5.

## Claims

1. An *in vitro* method for determining risk of an inflammatory condition in a subject, comprising
a. Detecting the level of VDAC1 in a biological sample from the subject,
b. Comparing the level in sample to control.

2. An *in vitro* method for determining risk of progression of an inflammatory condition in a subject determined to be at risk of, or diagnosed with, an inflammatory condition; said method comprising
a. Detecting the level of VDAC1 in a biological sample from the subject,
b. Comparing the level in sample to control.

3. The *in vitro* method according to claim 1 or 2 wherein a level of VDAC1 which is higher than control indicates increased risk for an inflammatory condition and/or progression of the inflammatory condition.

4. The *in vitro* method according to any of the preceding claims wherein determining risk comprises detecting the level of VDAC1 polypeptide in a biological sample.

5. The *in vitro* method according to any of the preceding claims wherein the biological sample is selected from the group comprising blood, serum, plasma and/or cell culture medium.

6. The *in vitro* method according to any of the preceding claims wherein the inflammatory condition is a chronic inflammatory disease or a condition with acute inflammatory component.

7. The *in vitro* method according to claim 6 wherein the chronic inflammatory disease is selected from the group consisting of rheumatoid arthritis, inflammatory bowel disease (such as Crohn's disease and ulcerative colitis), psoriasis, chronic obstructive pulmonary disease (COPD); cystic fibrosis, diabetes, such as e.g. Type I diabetes or Type II diabetes; kidney disease; neurodegenerative disease such as for example Alzheimer's; cardiovascular disease such as for example atherosclerotic plaque; cerebrovascular disease; autoimmune disease; and low-grade inflammation associated with aging.

8. The *in vitro* method according to according to claim 6 wherein the condition with acute inflammatory component is selected from the group consisting of infection, such as COVID 19; trauma; and sepsis.

9. The *in vitro* method according to any of the preceding claims 1 to 7 wherein the chronic inflammatory disease is Type I Diabetes.

10. The *in vitro* method according to any of the preceding claims, wherein detecting the level of VDAC1 comprises contacting the sample with an antigen-binding polypeptide having binding specificity for VDAC1, the polypeptide being selected from the group consisting of an antibody, an antigen-binding fragment thereof, and any variants, fusions, or derivatives thereof.

11. The *in vitro* method according to any of the preceding items wherein detecting the level of VDAC1 comprises an assay selected from the group consisting of lateral flow immunoassay (LFIA, aka dipstick test), immunochromatographic strip test, latex agglutination test (LAT), point-of-care immunosensors or biosensors, microfluidic-based rapid immunoassays, "lab-on-a-chip" devices integrating sample handling, mixing, and detection, flow-through immunoassay (e.g. dot-blot or spot test), immunoturbidimetric and nephelometric methods, snap tests (enzyme-linked immunosorbent assay in a cartridge) and enzyme-linked immunoassay (ELISA).

12. An antigen-binding polypeptide having binding specificity for VDAC1 for use in a diagnostic method practiced on the human or animal body, wherein the antigen-binding polypeptide is selected from the group consisting of an antibody, an antigen-binding fragment thereof, and any variants, fusions, or derivatives thereof.

13. An antigen-binding polypeptide having binding specificity for VDAC1 for use in the *in vitro* method according to claims 1 to 11, the polypeptide being selected from the group consisting of an antibody, an antigen-binding fragment thereof, and any variants, fusions, or derivatives thereof.

14. A compound for use in treatment of an inflammatory condition in a subject, wherein the subject is determined to be at risk for an inflammatory condition, or at risk for progression of an inflammatory condition, by the method according to any of the preceding claims 1 to 11.

15. A compound for use according to claim 14, wherein the inflammatory condition is Type I Diabetes, and wherein said compound is selected from one or more of the groups consisting of insulin (including insulin analogs such as insulin glargine, detemir, lispro, aspart, and degludec); a GLP-1 receptor agonist (such as exenatide, liraglutide, dulaglutide, or semaglutide); a SGLT2 inhibitor (such as canagliflozin, dapagliflozin, or empagliflozin); a DPP-4 inhibitor (such as sitagliptin, saxagliptin, linagliptin, or vildagliptin); a sulfonylurea (such as glibenclamide, glipizide, or glimepiride);a thiazolidinedione (such as pioglitazone or rosiglitazone); an immunomodulatory agent (such as an anti-CD3 antibody, interleukin inhibitor, or TNF-α inhibitor); blood pressure drugs; cholesterol lowering drugs; anti-platelet drugs (such as aspirin); and pharmaceutically acceptable salts thereof;

16. A method of determining a treatment regimen for Type I Diabetes comprising
a. performing the *in vitro* method of any of claims 1 to 11, and
b. determining a treatment protocol based on the determination
